(19) Europäisches Patentamt
European Patent Office
Office européen des brevets

(11) **EP 3 021 886 B1**

(12) **EUROPÄISCHE PATENTSCHRIFT**

(45) Veröffentlichungstag und Bekanntmachung des Hinweises auf die Patenterteilung:
**30.08.2023 Patentblatt 2023/35**

(21) Anmeldenummer: **14739431.6**

(22) Anmeldetag: **15.07.2014**

(51) Internationale Patentklassifikation (IPC):
***A61M 1/34*** *(2006.01)* ***A61M 1/36*** *(2006.01)*
***A61M 1/16*** *(2006.01)*

(52) Gemeinsame Patentklassifikation (CPC):
**A61M 1/34; A61M 1/341; A61M 1/36;
A61M 1/3643; A61M 1/3644; A61M 1/365;**
A61M 1/36224; A61M 1/362262; A61M 1/362264;
A61M 1/362265; A61M 2205/3331; A61M 2205/50

(86) Internationale Anmeldenummer:
**PCT/EP2014/065119**

(87) Internationale Veröffentlichungsnummer:
**WO 2015/007721 (22.01.2015 Gazette 2015/03)**

(54) **STEUER- ODER REGELVORRICHTUNG, KONFIGURIERT, UM EIN VERFAHREN ZUM STEUERN EINER BLUTBEHANDLUNGSVORRICHTUNG**

A CONTROL DEVICE CONFIGURED TO CONTROL A METHOD FOR CONTROLLING A BLOOD TREATMENT APPARATUS

DISPOSITIF DE PILOTAGE POUR CONTROLER UN PROCÉDÉ DE COMMANDE D'UN DISPOSITIF DE TRAITEMENT DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.07.2013 DE 102013011715**

(43) Veröffentlichungstag der Anmeldung:
**25.05.2016 Patentblatt 2016/21**

(73) Patentinhaber: **Fresenius Medical Care Deutschland GmbH
61352 Bad Homburg (DE)**

(72) Erfinder:
• **CHAMNEY, Paul
Tring
Hertfordshire HP23 5RU (GB)**
• **KOPPERSCHMIDT, Pascal
97456 Dittelbrunn (DE)**
• **WEHMEYER, Wolfgang
72076 Tübingen (DE)**

(74) Vertreter: **Bobbert & Partner
Patentanwälte PartmbB
Postfach 1252
85422 Erding (DE)**

(56) Entgegenhaltungen:
DE-A1-102009 018 664  DE-B4- 19 655 226
US-A- 4 670 152

• HÖRL WALTER H ET AL: "Passage, Dialyseverfahren in Klinik und Praxis : Technik und Klinik", 1 January 2004 (2004-01-01), DIALYSEVERFAHREN IN KLINIK UND PRAXIS : TECHNIK UND KLINIK, THIEME, DE, PAGE(S) 183 - 184, XP009505768, ISBN: 978-3-13-497706-6 * the whole document *

**Beschreibung**

Die vorliegende Erfindung betrifft eine Blutbehandlungsvorrichtung gemäß Anspruch 1.

Aus der Praxis sind Verfahren zum Steuern einer Blutbehandlungsvorrichtung bekannt, u. a. um einen extrakorporalen Blutschlauchsatz zu seiner Verwendung in einer Blutbehandlung, beispielsweise durch Primen oder Spülen desselben, vorzubereiten.

Die DE 10 2009 018 664 A1 offenbart eine externe Funktionseinrichtung, eine Blutbehandlungsvorrichtung zum Aufnehmen einer erfindungsgemäßen externen Funktionseinrichtung sowie Verfahren.

Die US 4,670,152 offenbart ein System zum Primen für eine Ultrafiltrationseinrichtung.

Die DE 196 55 226 B4 offenbart Verfahren zum Vorfüllen des Extrakorporalen Kreislaufs eines modularen Heimdialysesystems.

Eine Aufgabe der vorliegenden Erfindung ist es, eine Blutbehandlungsvorrichtung anzugeben.

Die erfindungsgemäße Aufgabe kann durch eine Blutbehandlungsvorrichtung gemäß Anspruch 1 gelöst werden.

Ein - vorzugsweise automatisches - Verfahren zum Steuern und/oder Regeln einer Blutbehandlungsvorrichtung ist offenbart, welche eine Druck- oder Unterdruckquelle - beispielsweise eine Blutpumpe zum Pumpen oder eine Ultrafiltratpumpe zum Saugen - alle dieser Alternativen, welche auch kombiniert eingesetzt werden können, werden im Folgenden auch verkürzt als Pumpen oder Blutpumpen bezeichnet, was nicht einschränkend sondern im hier dargelegten Sinn zu verstehen ist - zum Fördern von sich innerhalb eines extrakorporalen Blutkreislaufs befindenden Bluts aufweist. Der Blutschlauchsatz kann eines oder mehrere der folgenden Elemente aufweisen oder hiermit verbunden sein. So kann der Blutschlauchsatz einen arteriellen Abschnitt aufweisen, welcher eine arterielle Nadel aufweist oder hiermit verbunden ist, und welcher eine arterielle Patientenschlauchklemme aufweist. Der Blutschlauchsatz kann ferner einen venösen Abschnitt aufweisen, welcher eine venöse Nadel aufweist oder hiermit verbunden ist, und welcher eine venöse Patientenschlauchklemme aufweist. Zwischen dem arteriellen und dem venösen Abschnitt kann wenigstens ein Blutfilter angeordnet sein. Dieser weist eine Membran auf. Die Membran unterteilt eine Blutkammer von einer Dialysierflüssigkeitskammer.

Das offenbarte Verfahren umfasst ein Fördern eines Fluids (dieses kann Blut, ein Fluidgemisch, ein Gemisch wie Blutschaum oder eine andere Flüssigkeit mit Luft- oder Gasanteilen, Kochsalzlösung, Priminglösung oder dergleichen sein, weshalb der Begriff "Fluid" im Folgenden wie hier exemplarisch definiert zu verstehen ist) mittels der Blutpumpe bei geschlossener venöser Patientenschlauchklemme. Hierbei kann es sich um ein erstmaliges oder zu Beginn der Blutbehandlungssitzung erfolgendes Fördern handeln, oder um ein Fördern, bevor erstmals Blut durch den venösen Abschnitt oder durch die venöse Nadel Richtung Patient geströmt ist.

Der arterielle Abschnitt des Blutschlauchsatzes liegt stromaufwärts des Blutfilters. Der venöse Abschnitt des Blutschlauchsatzes liegt stromabwärts des Blutfilters. Die Bezeichnung "stromauf" oder "stromaufwärts" des Blutfilters bedeutet hierin bezogen auf den vorstehenden Satz, dass das extrakorporal strömende Blut bei üblicher Benutzung der Blutbehandlungsvorrichtung zunächst den arteriellen Abschnitt des Blutschlauchs durchströmt und erst anschließend den Blutfilter.

Die offenbarte Steuer- oder Regelvorrichtung ist eingerichtet, konfiguriert, bestimmt und/oder programmiert, um im Zusammenspiel mit einer mit den hierzu erforderlichen Einrichtungen ausgestatteten Blutbehandlungsvorrichtung das offenbarte Verfahren auszuführen oder sein Ausführen zu veranlassen.

Die erfindungsgemäße Blutbehandlungsvorrichtung weist eine Steuer- oder Regelvorrichtung auf oder ist hiermit verbunden und ist in Anspruch 1 beschrieben.

Das offenbarte Computerprogramm kann direkt in den internen Speicher eines digitalen Computers geladen werden und umfasst Softwarecodeabschnitte, mit denen die Schritte des Verfahrens ausgeführt werden oder ausführbar sind, wenn das Computerprogramm auf einem Computer läuft.

Das offenbarte digitale Speichermedium weist elektrisch auslesbare Steuersignale auf, die derart mit einem programmierbaren Computersystem oder Computer zusammenwirken können, dass die Schritte des Verfahrens veranlasst werden.

Ein offenbartes, insbesondere digitales, insbesondere nichtflüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart mit einem programmierbaren Computer oder Computersystem zusammenwirken, dass die maschinellen Schritte eines hierin beschriebenen Verfahrens veranlasst werden.

Dabei können alle, einige oder manche der maschinell durchgeführten Schritte des offenbarten Verfahrens veranlasst werden.

Ein offenbartes Computerprogramm-Produkt weist einen volatilen, flüchtigen oder auf einem maschinenlesbaren Träger oder Speichermedium gespeicherten Programmcode auf zur Veranlassung der Schritte des Verfahrens, wenn das Computerprogramm-Produkt auf einem Rechner abläuft. Unter einem Computerprogramm-Produkt kann beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Ein maschinenlesbarer Träger bezeichnet in bestimmten Ausführungsformen einen Träger, der mittels Software und/oder Hardware interpretierbare Daten oder Informationen enthält. Der Träger kann eine Diskette, eine CD, DVD, ein USB-Stick, eine Flashcard, eine SD-Karte und dergleichen sein.

Ein offenbartes Computerprogramm weist einen Programmcode auf zur Veranlassung der maschinellen Schritte eines Verfahrens, wenn das Computerprogramm auf einem Computer abläuft. Unter einem Computerprogramm kann beispielsweise ein physikalisches, vertriebsfähiges Software-Produkt verstanden werden, welches ein Programm aufweist.

Auch für das offenbarte Computerprogramm-Produkt und das Computerprogramm gilt, dass alle, einige oder manche der maschinell durchgeführten Schritte des Verfahrens veranlasst werden.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Während des Förderns befindet sich in manchen erfindungsgemäßen Ausführungsformen im venösen Abschnitt vollständig oder teilweise ein anderes Fluid als Blut, beispielsweise eine Priminglösung. Eine Priminglösung kann eine Kochsalzflüssigkeit, eine aufbereitete Dialysierflüssigkeit, eine Mischung aus den genannten Flüssigkeiten oder andere Fluide enthalten oder hieraus bestehen.

In manchen erfindungsgemäßen Ausführungsformen wird unter einer venösen Patientenschlauchklemme, welche ganz oder teilweise geschlossen ist, auch eine Patientenschlauchklemme verstanden, welche eine merkliche Drosselung des Stroms durch den venösen Abschnitt derart bewirkt, dass aufgrund der Pumpwirkung der Blutpumpe Fluid durch die Membran des stromauf der Patientenschlauchklemme liegenden Blutfilters hindurch tritt. In anderen erfindungsgemäßen Ausführungsformen erlaubt eine geschlossene venöse Patientenschlauchklemme keinen Fluidstrom über sie hinweg.

Bei der venösen Patientenschlauchklemme handelt es sich in manchen erfindungsgemäßen Ausführungsformen um eine beliebige Einrichtung zum Unterbrechen oder Drosseln des Flusses im venösen Abschnitt des Blutschlauchsatzes stromab des Blutfilters.

Bei der arteriellen Patientenschlauchklemme handelt es sich in einigen erfindungsgemäßen Ausführungsformen um jede Einrichtung zum Unterbrechen oder Drosseln des Flusses im arteriellen Abschnitt des Blutschlauchsatzes stromauf des Blutfilters.

In bestimmten erfindungsgemäßen Ausführungsformen wird Fluid solange gefördert oder gesaugt, beispielsweise mittels der Dialysat- und/oder der Ultrafiltrationspumpe, bis - vorzugsweise mindestens oder höchstens - ein vorbestimmtes Volumen von Fluid durch die Membran hindurch aus dem Blutschlauchsatz, insbesondere aus dessen arteriellen Abschnitt, in die Dialysierflüssigkeitskammer übergetreten ist.

Das Fluid, welches als vorbestimmtes Volumen durch die Membran hindurch in die Dialysierflüssigkeitskammer übertritt, kann als Ultrafiltrat bezeichnet werden.

Das vorbestimmte Volumen, welches über die Membran hindurch tritt, kann sich in einigen erfindungsgemäßen Ausführungsformen ergeben aus einer vorbestimmten Zeitdauer, in welcher die Blutpumpe mit einer vorbestimmten Förderrate fördert.

Im Folgenden wird für den Begriff "Patientenschlauchklemme" alternativ auch der Begriff "Schlauchklemme" verwendet.

In bestimmten erfindungsgemäßen Ausführungsformen wird das Fluid von der Blutpumpe bei geschlossener venöser Schlauchklemme solange als Ultrafiltrat durch die Membran hindurch aus dem Blutschlauchsatz in die Dialysierflüssigkeitskammer gefördert, bis wenigstens ein Sensorsignal das Eintreffen von Blut an einer vorbestimmten Stelle detektiert, anzeigt oder hierfür codiert.

Eine "vorbestimmte Stelle" kann am oder im Blutfilter (in der Blutkammer oder in der Dialysierflüssigkeitskammer) sein. Die "vorbestimmte Stelle" kann jedoch auch stromauf oder stromab (stromab nur sofern die venöse Patientenschlauchklemme nicht vollständig geschlossen ist) der Blutkammer, auf der Blutseite oder auf der Dialysatseite sein. Die Dialysatseite schließt sich stromauf und stromab der Dialysierflüssigkeitskammer an.

Das Sensorsignal ist in einigen erfindungsgemäßen Ausführungsformen ein Drucksignal.

In gewissen erfindungsgemäßem Ausführungsformen wird der Zeitraum zwischen Beginn der Förderung und erstem Detektieren von Blut mittels des Sensorsignals als erste Phase oder Phase 1 bezeichnet.

In bestimmten erfindungsgemäßen Ausführungsformen wird das Fördern von Fluid reduziert oder gestoppt, nachdem ein vorbestimmtes Volumen von Fluid in die Dialysierflüssigkeitskammer übergetreten ist, oder wenigstens ein Sensorsignal das Erreichen von Blut an der vorbestimmten Stelle detektiert wird. Diese Phase, in der das Fördern von Fluid reduziert oder gestoppt wird, wird im Folgenden als zweite Phase oder Phase 2 bezeichnet.

In einigen erfindungsgemäßen Ausführungsformen vermischt sich in der zweiten Phase das geförderte Fluid zunehmend mit dem Fluid nachfolgend geförderten Blut. Mit zunehmender Förderdauer kann sich der Anteil von Blut gegenüber dem Anteil von Fluid erhöhen. In der zweiten Phase kann der Anteil von Fluid, welcher als Ultrafiltrat durch die Membran hindurch aus dem Blutschlauchsatz in die Dialysierflüssigkeitskammer übertritt, verringert werden. Um dies bei gleichbleibender oder annähernd gleichbleibender Förderrate der Blutpumpe, welche in der zweiten Phase das Gemisch aus Fluiden, insbesondere das Gemisch aus Primingfluid und Blut, fördert, zu erreichen, wird in einigen erfindungsgemäßen Ausführungsformen daher die venöse Schlauchklemme ganz oder teilweise geöffnet. Auf diese Weise fließt zumindest ein Teil des geförderten Volumens durch den venösen Abschnitt. Alternativ oder ergänzend kann die Förderleistung der Pumpe geeignet gesenkt werden.

Das Verringern der Förderung von Fluid, welches als Ultrafiltrat durch die Membran hindurch aus dem Blutschlauchsatz in die Dialysierflüssigkeitskammer übertritt, erfolgt in gewissen erfindungsgemäßen Ausführungsformen mit zunehmender Zeitdauer linear oder nicht linear, z. B. progressiv oder degressiv.

In einigen erfindungsgemäßen Ausführungsformen basiert das Drucksignal, welches als Sensorsignal das Erreichen von Blut an der vorbestimmten Stelle detektiert, auf Druckpulsen, die von der Blutpumpe ausgehen oder fortgeleitet werden. Diese Druckpulse werden durch das geförderte Volumen (Fluid und/oder Blut) oder mittels diesem oder entlang diesem weitergeleitet und können transversal Poren der Membran des Blutfilters durchlaufen.

Die Druckpulse können beispielsweise auf der Seite der Dialysierflüssigkeitskammer oder in den zu- oder abführenden Leitungen der Dialysierflüssigkeitskammer, also auf der hydraulischen Seite, mittels wenigstens eines hierzu vorgesehenen Drucksensors detektiert und analysiert werden.

Eine Einrichtung, welche eingerichtet, konfiguriert, vorbereitet und/oder programmiert ist zum Auswerten, Erkennen, oder Vergleichen derartiger Drucksignale ist in manchen erfindungsgemäßen Ausführungsformen vorgesehen.

Die Drucksensoren zum Detektieren und Analysieren der Druckpulse sind in gewissen erfindungsgemäßen Ausführungsformen Drucksensoren, die in der Blutbehandlungsvorrichtung zum Messen oder Bestimmen von arteriellen oder venösen Drücken eingesetzt werden oder hierzu vorgesehen sind.

Die mittels des Drucksensors detektierten Amplituden der Druckpulse werden in gewissen erfindungsgemäßen Ausführungsformen analysiert. Diese Druckamplituden, die in gewissen Zeitabständen von der Blutpumpe erzeugt und ausgesendet werden, beispielsweise basierend auf den Rolleneingriffen von Rollerpumpen als Blutpumpen, können sich im zeitlichen Verlauf verändern. Im Folgenden werden als Ausgangsamplituden beispielsweise die mittels Drucksensoren in der Dialysierflüssigkeitskammer (oder deren zu- oder abführenden Leitungen) detektierten Druckamplituden bezeichnet, als Eingangsamplituden beispielsweise die mittels Drucksensoren im oder am Blutschlauchsatz, vorzugsweise zwischen Blutpumpe und Blutfilter detektierten Druckamplituden. In der ersten Phase (Phase 1) kann sich ein festes Verhältnis zwischen Ausgangs- zu Eingangsamplitude, bezogen auf die Blutkammer, einstellen. Nimmt die Dämpfung der Druckpulse im Fluid zu, d.h. die Ausgangsamplitude nimmt gegenüber der Eingangsamplitude ab, kann dies ein Indiz für den Beginn einer Vermischung von Fluid mit nachfolgendem Blut sein. Der Beginn der Vermischung von Fluid mit Blut kann das Ende der Phase 1 oder den Beginn einer nachfolgenden Phase 2 kennzeichnen.

In einigen erfindungsgemäßen Ausführungsformen wird die Verringerung oder Reduktion von Fluid, welches durch die Membran in die Dialysierflüssigkeitskammer übertritt (dieser Volumenstrom wird im Folgenden auch als Ultrafiltratfluss bezeichnet) mittels der Ausgangsamplituden des entsprechenden Drucksensors (siehe oben) bestimmt oder ermittelt.

Sollten die Eingangsamplituden (siehe oben) nicht bekannt sein, so werden in bestimmten erfindungsgemäßen Ausführungsformen beispielsweise allein die Ausgangsamplituden als Startpunkt und/oder als Maß für die - insbesondere erforderliche - Reduktion des Ultrafiltratflusses, insbesondere wie vorstehend erläutert, genutzt.

Für die Bestimmung des Ultrafiltratflusses werden in einigen erfindungsgemäßen Ausführungsformen - vorzugsweise allein - die Ausgangsamplituden, beispielsweise als Modulationsbreite $A_{out}$, der Druckschwankungen im Fluid am Drucksensor, insbesondere der Ausgangsamplituden, detektiert.

Der Ultrafiltratfluss kann mittels der folgenden Gleichung bzw. Formel ermittelt werden:

$$Q_{UF}(t) \propto \alpha \cdot Q_B \cdot \frac{A_{out}(t)}{A_{out}(t=0)} \qquad (1)$$

mit:

$Q_{UF}$ (t) :  zeitlicher Verlauf des Ultrafiltratflusses durch die Membran;

$Q_B$:  Blutfluss;

$\alpha$:  Faktor, welcher angibt, welcher Anteil vom Blutfluss $Q_B$ in der Phase 2 über die Membran abfiltriert werden soll;

$A_{out}(t)$:  Modulationsbreite von Druckschwankungen der Druckpulse zum Zeitpunkt t; und

$A_{out}(t=0)$:  Modulationsbreite von Druckschwankungen der Druckpulse zum Zeitpunkt t=0; der Zeitpunkt t=0 kann der Beginn der Betrachtung und/oder des erfindungsgemäßen Verfahrens sein.

Mittels der Formel (1) kann das Maß bestimmt werden, um welches die Ultrafiltration verringert werden soll. Dieses hängt in gewissen erfindungsgemäßen Ausführungsformen vom zunehmenden Anteil an Blut am geförderten Fluid ab.

$Q_{UF}$ (t) wird in einigen erfindungsgemäßen Ausführungsformen an der Ultrafiltrationspumpe eingestellt.

$Q_B$ wird in manchen erfindungsgemäßen Ausführungsformen an der Blutpumpe eingestellt.

In bestimmten erfindungsgemäßen Ausführungsformen ist das Sensorsignal ein optisches Signal.

Das optische Signal wird mittels dem Fachmann bekannter optischer Sensoren detektiert. Mit Hilfe eines optischen Sensors, der beispielsweise das Fluid und/oder das Blut mittels einer Farbänderung - beispielsweise in der Blutkammer oder an anderer Stelle - detektieren kann, ist es möglich, den Beginn der zweiten Phase zu bestimmen oder zu erkennen. Das Detektieren des Beginns der zweiten Phase kann ein Reduzieren oder Stoppen des Förderns von Fluid und/oder Blut zur Folge haben, beispielsweise initiiert mittels einer Steuer- oder Regeleinrichtung der erfindungsgemäßen Blutbehandlungsvorrichtung.

In manchen erfindungsgemäßen Ausführungsformen entspricht die vorbestimmte Menge an Volumen ganz, teilweise, im Wesentlichen oder zu einem vorbestimmten Anteil jenem Volumen der Priminglösung, welches mittels der Blutpumpe aus dem arteriellen Abschnitt durch die Membran hindurch in die Dialysierflüssigkeitskammer übergetreten ist.

Unter einer Priminglösung wird erfindungsgemäß jede Flüssigkeit verstanden, welche zum Befüllen und/oder Spülen des Blutschlauchsatzes vor Beginn der Blutbehandlung verwendet wird. Üblicherweise handelt es sich um Dialysierflüssigkeit oder Substituierflüssigkeit. Bei der Priminglösung kann es sich um eine sterile, physiologisch verträgliche Lösung handeln.

In bestimmten erfindungsgemäßen Ausführungsformen entspricht die vorbestimmte Menge an Volumen einem Gesamtfassungsvermögen des Blutschlauchsatzes oder einem Abschnitt hiervon. Das Gesamtfassungsvermögen kann beispielsweise dem Fassungsvermögen der Leitungen des Blutschlauchsatzes ohne Blutfilter, oder mit Blutfilter, oder nur mit der Blutkammer des Blutfilters, entsprechen. Als Abschnitt des Blutschlauchsatzes kommt der arterielle Abschnitt ohne Blutfilter, oder mit Blutfilter, oder nur mit der Blutkammer des Blutfilters, in Frage.

In manchen erfindungsgemäßen Ausführungsformen umfasst das Verfahren ein Ausgeben oder Absenden eines Steuerbefehls an die Blutbehandlungsvorrichtung oder an einen Datenempfänger, wobei der Steuerbefehl geeignet ist, die Blutbehandlungsvorrichtung zu veranlassen, in einem ersten Zeitraum mit einer höheren Ultrafiltrationsrate zu filtrieren - oder diese entsprechend eingestellt ist, um mit einer höheren Ultrafiltrationsrate zu filtrieren - als in einem nach dem ersten Zeitraum gelegenen zweiten Zeitraum.

In einigen erfindungsgemäßen Ausführungsformen umfasst das Verfahren ein Ausgeben eines Steuerbefehls an die Blutbehandlungsvorrichtung derart oder mit der Wirkung, dass diese in einem, sich einem wie vorstehend beschriebenen Verfahren zeitlich mittelbar oder unmittelbar anschließenden, ersten Zeitraum mit einer höheren Ultrafiltrationsrate filtriert als in einem nach dem ersten Zeitraum gelegenen zweiten Zeitraum, wobei die venöse Patientenschlauchklemme im ersten und im zweiten Zeitraum geöffnet ist.

Mit dem Begriff "Öffnen" der venösen und/oder arteriellen Patientenschlauchklemme ist in einigen erfindungsgemäßen Ausführungsformen ein vollständiges oder teilweises Öffnen der Patientenschlauchklemme gemeint, um einen teilweisen oder vollständigen Durchfluss von Fluid und/oder Blut durch den venösen Patientenschlauch oder Blutschlauchabschnitt hindurch zu ermöglichen. Das Öffnen kann manuell oder automatisch geschehen.

Die höhere Ultrafiltrationsrate kann in einigen erfindungsgemäßen Ausführungsformen vorteilhaft dazu dienen, ein mögliches Restvolumen von Fluid als Primingvolumen im Blutschlauchsatz während der Behandlungszeit zusätzlich zu filtrieren.

Die während der Behandlungszeit - sei es während der gesamten Behandlungszeit oder nur während eines Abschnitts hiervon, z. B. während des ersten Zeitraums - angelegte - konstante oder nicht-konstante - Ultrafiltrationsrate, und insbesondere die vorstehend als höher bezeichnete Ultrafiltrationsrate, kann derart festgelegt sein, dass sie zusätzlich zu dem zur Behandlung des Patienten vorgesehenen Ultrafiltrationsvolumen auch die Ultrafiltration eines Volumens bewirkt, welches dem möglichen Restvolumen von Fluid als Primingvolumen im Blutschlauchsatz entspricht.

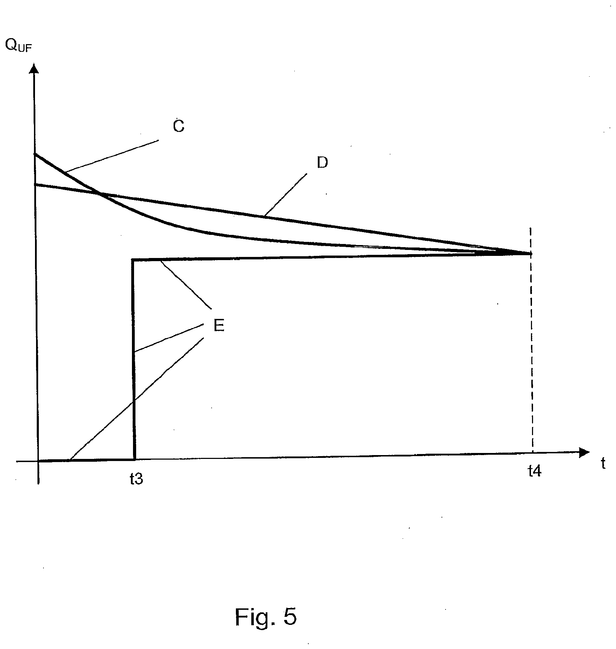

Der erste Zeitraum liegt in bestimmten erfindungsgemäßen Ausführungsformen zu Beginn der Blutbehandlungszeit, der zweite Zeitraum beginnt und endet nach Ende des ersten Zeitraums. Mögliche Ausführungsformen werden in Fig. 5 dargestellt. Zu ihnen zählen beispielsweise ein linearer Abfall der Ultrafiltrationsrate, ein progressiver Abfall, ein degressiver Abfall, ein kontinuierlicher Abfall, ein stetiger Abfall, oder Kombinationen hieraus.

In manchen erfindungsgemäßen Ausführungsformen zählt zur Blutbehandlung nicht das Befüllen des extrakorporalen Blutkreislaufs und/oder nicht jene Zeit, die bis zum Öffnen der venösen Schlauchklemme vergeht.

Zur Blutbehandlungszeit zählt in bestimmten erfindungsgemäßen Ausführungsformen allein jene Zeit, während welcher das Blut durch Kontakt mit dem Blutfilter gereinigt, gefiltert oder anderweitig behandelt wird. Das Befüllen des extrakorporalen Blutkreislaufs und/oder nicht jene Zeit, die bis zum Öffnen der venösen Schlauchklemme vergeht, zählt in manchen erfindungsgemäßen Ausführungsformen nicht zur Blutbehandlungszeit.

In gewissen erfindungsgemäßen Ausführungsformen fällt die Ultrafiltrationsrate sowohl im ersten als auch im zweiten Zeitraum ausschließlich ab. In bestimmten erfindungsgemäßen Ausführungsformen fällt die Ultrafiltrationsrate über die gesamte Blutbehandlungsdauer ausschließlich ab.

In einigen erfindungsgemäßen Ausführungsformen bewirkt der Steuerbefehl oder kodiert hierfür, dass die Ultrafiltrationsrate innerhalb des ersten Zeitraums degressiv abnimmt. In gewissen erfindungsgemäßen Ausführungsformen ist der Steuerbefehl geeignet, die Ultrafiltrationsrate innerhalb des ersten Zeitraums abfallen zu lassen. In bestimmten erfindungsgemäßen Ausführungsformen ist die Ultrafiltrationsrate bei Empfang des Steuerbefehls eingestellt, um innerhalb des ersten Zeitraums ungeachtet von Messergebnissen von Sensoren abzufallen.

In manchen erfindungsgemäßen Ausführungsformen bewirkt der Steuerbefehl, dass im ersten Zeitraum ein Volumen über die Membran hinweg filtriert wird, welches - beispielsweise wenigstens oder höchstens - um das Fassungsvolumen der Blutkammer des Blutschlauchsets und des hiervon stromabwärts gelegenen Blutschlauchsatzes höher ist als ein Volumen, welches im zweiten Zeitraum über die Membran hinweg filtriert wird.

Der erste und der zweite Zeitraum sind in einigen erfindungsgemäßen Ausführungsformen gleich lang.

In bestimmten erfindungsgemäßen Ausführungsformen, bei welchen es sich um ein eigenständiges Verfahren handeln kann, welches keine Merkmale der vorstehend beschriebenen Ausführungsformen und/oder Verfahren aufweisen muss, sondern für sich allein stehen und in Form von entsprechenden Patentansprüchen auch für sich allein beansprucht werden kann, wird die Ultrafiltrationsrate während der Behandlungssitzung zusätzlich erhöht, zum Kompensieren eines gegen Ende der Behandlungssitzung zusätzlich zuzuführenden Volumens (z. B. eine Kochsalzlösung, zugeführt als Infusion) in das Gefäßsystem des Patienten. Das zusätzlich zuzuführende Volumen kann einem Volumenausgleich, welcher nach dem Ende der Behandlungssitzung innerhalb der beiden Flüssigkeitskompartimente des Patienten, dem extrazellulären Raum (im Folgenden als ECM abgekürzt; ECM - extracellular matrix) und den Blutgefäßen, eintritt, ganz oder teilweise entgegenwirken oder diesen reduzieren.

In einigen erfindungsgemäßen Ausführungsformen wird das zusätzlich zuzuführende Volumen mittels Bestimmung eines Parameters festgelegt, welcher ein Maß für die Änderung des Plasmawasser-Anteils im Blut des Patienten ist und/oder erfindungsgemäß als ein solches Maß ausgewertet wird. Mögliche Parameter hierfür sind in gewissen erfindungsgemäßen Ausführungsformen der Hämatokrit, der Hämoglobinwert oder (andere) Viskositätsparameter des Bluts. Einrichtungen zum Messen solcher Parameter können erfindungsgemäß vorgesehen sein. So kann insbesondere eine Einrichtung zum Messen des Hämatokrits vorgesehen sein. Diese kann rein exemplarisch ausgestaltet sein, um den Hämatokrit mittels optischer Messung zu messen, oder auf andere Weise.

Die Bestimmung des Parameters erfolgt in manchen erfindungsgemäßen Ausführungsformen zu wenigstens zwei Zeitpunkten während der Behandlungssitzung und vorzugsweise am Blut im extrakorporalen Blutschlauchsatz. Für die Parameterbestimmung kann dem extrakorporalen Blutschlauchsatz Blut entnommen werden.

In einigen erfindungsgemäßen Ausführungsformen umfasst das Verfahren ein Ausgeben eines Steuerbefehls an die Blutbehandlungsvorrichtung derart, dass diese zwischen zwei Zeitpunkten während der Behandlungssitzung mit einer erhöhten ersten Ultrafiltrationsrate filtriert, die das gegen Ende der Behandlungssitzung zusätzlich zugeführte Volumen - sozusagen bereits bevor dieses zugeführt wurde - kompensiert.

In bestimmten erfindungsgemäßen Ausführungsformen wird zu Beginn der Behandlungssitzung ein Referenzwert gebildet oder erstellt, der als eine Bezugsgröße für den angestrebten Plasmawasser-Anteil im Blut des Patienten am Ende der Behandlungssitzung dient oder bestimmt wird.

Diese Bezugsgröße kann die folgenden drei Volumina beinhalten oder hieraus bestehen: das Verteilungsvolumen $V_{pat}$ (aus dem Verteilungsvolumen stammt das *refilling water,* das zum bekannten *refilling* führt), das Blutvolumen $V_{Blut}$ (Blutmenge im Blutkompartment) des Patienten, und das Volumen, das dem Blutvolumen zusätzlich mittels Ultrafiltration entzogen werden soll, abgekürzt als $V_{UF}$.

In bestimmten erfindungsgemäßen Ausführungsformen wird vereinfacht das Volumen des extrazellulären Raums ECM als tatsächlich gleich dem Verteilungsvolumen Vpat angenommen. Vpat kann beispielsweise mittels der Watson-Formel oder mittels des sogenannten "*Body Composition Monitors"* (BCM) ermittelt werden, wie er von der Fa. Fresenius Medical Care, Deutschland, vertrieben wird, oder wie er beispielsweise in der internationalen Patentanmeldung WO 2006/002685 A1 offenbart ist. Die Watson-Formel ist eine gebräuchliche, empirische, zugeschnittenen Größengleichung, bei der nach Eingabe bzw. unter Berücksichtigung definierter Parameter wie Größe, Gewicht, Geschlecht der Wasserbestand des Körpers errechnet wird. Die Watson-Formel ist in Rechnern von Dialysegeräten hinterlegt und Grundlage für die Kt/V-Bestimmung. Beispielsweise gibt Schönweiß (Schönweiß, Günther; Dialysefibel 3, Band 2, dritte, völlig neu bearbeitete und erweiterte Auflage, abakiss Verlagsgesellschaft mbH, 2006, S. 481-482) als Harnstoffverteilungsraumes V (in Liter), das dem Körperwasser entspricht, an:

Männer: 2,45 -[0,09 x Alter in Jahren + 0,11 x Größe in cm + 0,34 x Gewicht in kg]

Frauen: - 2,1 + [0,11 x Größe in cm + 0,25 x Gewicht in kg]

Diese zugeschnittene Größengleichung findet sich so auch bei Hörl und Wanner (Hörl, W. H.; Wanner C., Dialyseverfahren in Klinik und Praxis, 6., völlig neu bearbeitete Auflage; Georg Thieme Verlag, 2003, Seite 210).
Das Harnstoffverteilungsvolumen kann alternativ mittels Bioimpedanzmessung (BCM "Body Composition Monitor") ermittelt werden.
Somit kann das Volumen zu Beginn der Behandlungssitzung als Bezugsgröße bestimmt werden zu:

$$V_{ges,\,Beginn} = V_{pat} + V_{Blut} + V_{UF} \qquad (2)$$

Das Volumen am Ende der Behandlungssitzung kann bestimmt werden zu:

$$V_{ges,\,Ende} = V_{pat} + V_{Blut} \qquad (3)$$

Der Plasmawasser-Anteil im Blut ist zu Beginn der Behandlung aufgrund der noch nicht erfolgten oder begonnenen Ultrafiltration gegenüber dem Zeitpunkt nach Ultrafiltration um den Faktor x erhöht:

$$x = \frac{V_{pat} + V_{Blut} + V_{UF}}{V_{pat} + V_{Blut}} \qquad (4)$$

In einigen erfindungsgemäßen Ausführungsformen kann der Anteil aller zellulären Bestandteile am Volumen des Blutes (angegeben in Prozent; 100% = 1; im Folgenden als Hämatokrit bezeichnet) zur Bestimmung des Plasmawasser-Anteils im Blut verwendet werden, der wie folgt bestimmt werden kann:

$$V_{Plasma,\,ges} = V_{ges}\left(1 - HKT\right) \qquad (5)$$

Dabei wird der Gesamteiweiß-Beitrag des Blutes in diesem Ausführungsbeispiel nicht berücksichtigt.
Folglich besteht zwischen dem Hämatokrit zu Beginn und bei Ende der Behandlungssitzung folgender Zusammenhang, sofern das ECM zu dem Blutvolumen im Gleichgewicht steht (es findet kein sogenannter kein Refilling-Prozess mehr statt):

$$\left(1 - HKT_{Beginn}\right)\left(V_{pat} + V_{Blut} + V_{UF}\right) = \left(1 - HKT_{Ende}\right)\left(V_{pat} + V_{Blut}\right) \qquad (6)$$

Das zusätzlich zugeführte Volumen in das Blutgefäß des Patienten (Infusionsvolumen) ist in einigen erfindungsgemäßen Ausführungsformen daher so zu bestimmen, dass am Ende der Behandlungssitzung gilt:

$$HKT_{Ende} = 1 - \left(1 - HKT_{Beginn}\right)\left(\frac{V_{pat} + V_{Blut} + V_{UF}}{V_{pat} + V_{Blut}}\right) \qquad (7)$$

Das Volumen $V_{pat}$ kann als Verteilungsraum ohne Blutvolumen und ohne Überwässerung angenommen werden.
In gewissen erfindungsgemäßen Ausführungsformen wird zu Beginn der Behandlungssitzung der Hämatokrit oder ein Plasmawasser-Anteil im Blut als ein Referenzwert bestimmt. Ferner wird dem Patienten gegen Ende der Behandlungssitzung das zusätzliche Volumen zugegeben, beispielsweise intravenös oder, bevorzugt, über den extrakorporalen Blutkreislauf. Die Ultrafiltration wird derart geregelt oder gesteuert, dass der Hämatokrit oder der Plasmawasser-Anteil im Blut bei Ende der Behandlungssitzung einen zuvor festgelegten Wert annimmt und/oder in einem zuvor festgelegten Wertebereich liegt.

Der Hämatokrit oder der Plasmawasser-Anteil im Blut bei Ende der Behandlungssitzung liegt in einigen erfindungsgemäßen Ausführungsformen höher als jener, welcher zu Beginn gemessen wurde.

Der Hämatokrit oder der Plasmawasser-Anteil im Blut liegt in manchen erfindungsgemäßen Ausführungsformen bei Ende der Behandlungssitzung beispielsweise um 5 bis 25%, beispielsweise um 5 bis 15%, oder beispielweise - vorzugsweise etwa oder genau - 10% höher als zu Beginn der Behandlungssitzung.

Der Hämatokrit oder der Plasmawasser-Anteil im Blut liegt in manchen erfindungsgemäßen Ausführungsformen bei Ende der Behandlungssitzung beispielsweise um den Quotienten aus Überwässerung (messbar durch den hierin genannten Blood Composition Monitor, BCM, oder andere, dem Fachmann geeignete Verfahren und Vorrichtungen) und Verteilungsvolumen höher als zu Beginn der Behandlungssitzung. Das Verteilungsvolumen kann dem hier als $V_{pat}$ bezeichneten Volumen entsprechen.

In einigen erfindungsgemäßen Ausführungsformen ist die Behandlungsvorrichtung und/oder eine Steuereinrichtung entsprechend eingerichtet, konfiguriert und/oder programmiert, um das Verfahren auszuführen.

Da das zusätzlich zugeführte Volumen folglich erst am Ende der Behandlungssitzung bestimmt werden kann, dieses jedoch zur Wahrung einer Gesamtvolumenbilanz dem Patienten bereits während der Behandlungssitzung mittels Ultrafiltration entzogen werden soll, kann das zusätzlich zuzuführende Volumen beispielsweise über eine oder mehrere Behandlungssitzungen hinweg iterativ bestimmt, erlernt oder approximiert sein oder werden, oder anhand von Tabellen bestimmt werden.

In bestimmten erfindungsgemäßen Ausführungsformen wird das zusätzlich zuzuführende Volumen basierend auf einer Ermittlung des absoluten Blutvolumens des Patienten bestimmt. Das absolute Blutvolumen des Patienten kann entweder mittels einer Gewichtsabschätzung des Patienten oder einer Abschätzung basierend auf Daten aus einer Messung mittels eines sogenannten "*Body Composition Monitors*" (BCM) ermittelt werden, wie er von der Fa. Fresenius Medical Care Deutschland, vertrieben wird, oder wie er beispielsweise in der internationalen Patentanmeldung WO 2006/002685 A1 offenbart ist. Die Abschätzung mittels BCM basiert auf Daten des extrazellulären Raums ECM (synonym zu ECW - extracellular water) und des intrazellulären Raum ICM (synonym zu ICW - Intracellular Water).

Ein Steuerbefehl ist in manchen erfindungsgemäßen Ausführungsformen ein maschinenlesbarer oder maschinenausführbarer Befehl oder eine (z. B. noch zu kompilierende) Vorstufe hiervon. Er kann ein Signal sein.

In bestimmten erfindungsgemäßen Ausführungsformen ist die Blutbehandlungsvorrichtung zur Akutdialyse, zur chronischen Dialyse, oder zur Aquapherese, insbesondere als sog. SCUF-Gerät (SCUF = *slow continuous ultrafiltration*) ausgestaltet.

Jede der hierin genannten Einrichtungen und Vorrichtungen der erfindungsgemäßen Vorrichtung kann eigens eingerichtet, konfiguriert, angesteuert und/oder programmiert oder auf andere Weise vorbereitet sein, um den jeweiligen Verfahrensschritt auszuführen, auch wenn dies nicht eigens hierin ausgeführt ist.

In einigen erfindungsgemäßen Ausführungsformen wird kein Vakuum an den Ausgang der Dialysierflüssigkeitskammer des Blutfilters angelegt und/oder erzeugt.

In manchen erfindungsgemäßen Ausführungsformen ist die Blutbehandlungsvorrichtung nicht zur Blutbehandlung während einer Herz-Bypass-Operation, insbesondere zur Sauerstoffanreicherung, ausgestaltet.

In einigen erfindungsgemäßen Ausführungsformen ist wenigstens der Blutfilter als nicht wieder verwendbarer und/oder nicht wieder aufbereitbarer Filter, ausgestaltet und/oder vorgesehen bzw. wird nur einmalig verwendet. Alternativ oder zusätzlich können weitere Teile des Blutschlauchsatzes oder des extrakorporalen Blutkreislaufs auf diese Art und Weise, beispielsweise als Einweg-Blutkassette, ausgestaltet und/oder vorgesehen sein.

In manchen erfindungsgemäßen Ausführungsformen weist das Verfahren als Verfahrenschritt das Befördern von Fluid mittels der Ultrafiltrationspumpe ausschließlich in Richtung zum Blutfilter auf.

Die zum offenbarten Verfahren getroffenen Feststellungen und Definitionen treffen immer dort, wo dies für den Fachmann zu keinem Widerspruch führt, auch auf die erfindungsgemäßen Vorrichtungen zu, und umgekehrt.

Manche oder alle erfindungsgemäßen Ausführungsformen können einen, mehrere oder alle der oben und/oder im Folgenden genannten Vorteile aufweisen.

Das vorliegende Verfahren kann eine Verbesserung des bekannten *synchronuous connection* Verfahrens darstellen, welches das Verbinden des Patienten mit dem Blutschlauchsatz vor Beginn der Blutbehandlung betrifft. Bei diesem bekannten Verfahren werden die arterielle Patientennadel und die venöse Patientennadel gleichzeitig mit dem Gefäßsystem des Patienten, beispielsweise der Fistel, verbunden. Das gesamte Füllvolumen (z. B. die Priminglösung), welches sich vom vorangegangenen Befüllen und Primen des Blutschlauchsatzes noch in diesem befindet, wird über die venöse Patientennadel dem Gefäßsystem des Patienten zugeführt. Dies geschieht mittels der Blutpumpe, welche durch Ansaugen von Blut die Priminglösung vor sich her bis in die venöse Patientennadel und über diese in das Gefäßsystem treibt. Auf diese Weise gelangt das zur Füllung des gesamten Blutschlauchsatzes erforderliche Volumen der Priminglösung in das Blutsystem des Patienten. Das zur Füllung des Blutschlauchsatzes erforderliche Volumen der Priminglösung geht damit in die Flüssigkeitsbilanz der Behandlungssitzung mit ein, was eine Korrektur der Bilanz durch Justierung der (gewünschten) Gesamtgewichtsabnahme des Patienten an der Dialysevorrichtung erfordert. Ein derartige Korrektur, welche durch das Personal vorgenommen wird, was allerdings gelegentlich vergessen wird, kann jedoch die komplexen und dynamischen Effekte wie den *water rebound* nicht berücksichtigen. Diese Effekte ergeben sich durch das sog. *Refilling* von Flüssigkeit, welches auch noch nach Beendigung der Blutbehandlung aus dem extravaskulären Raum in die Blutgefäße einströmt.

Die vorliegende Erfindung verringert die vorstehend beschriebene, erforderliche Korrektur durch das Personal und die hiermit einhergehenden Unzulänglichkeiten, welche aufgrund von *refilling* Prozessen entstehen könnten, welche wiederum Anlass zur Korrektur der Flüssigkeitsbilanz sind. Dies erfolgt dadurch, dass erfindungsgemäß bereits vor dem Beginn der Behandlung des Blutes ein Großteil des zur Füllung des Blutschlauchsatzes erforderlichen Volumens der Priminglösung vorteilhaft aus dem Blutschlauchsatz entfernt wird und gar nicht erst in das Blutgefäß des Patienten und damit in die Flüssigkeitsbilanz gelangt.

Zudem kann eine Priminglösung, welche nicht in das Gefäßsystem des Patienten gelangt, dessen onkotischen Druck nicht senken - ein Einströmen von Fluid in den extravaskulären Raum aufgrund des abgesenkten onkotischen Drucks unterbleibt vorteilhaft. Damit kann die sich anschließende Blutbehandlung früher beendet werden oder bei einer höher eingestellten Ultrafiltrationsrate erfolgen; ein *refilling* des aufgrund des niedrigen onkotischen Drucks in den extravaskulären Raum übergetretenen Fluids bleibt vorteilhaft aus. Eine "vorauseilende" Ultrafiltration, mit welcher dieses *refilling* bereits a priori ausgeglichen werden könnte, ist nicht erforderlich. Ein mit der vorauseilenden Ultrafiltration einhergehendes Risiko der Hypotonie des Patienten kann erfindungsgemäß vorteilhaft vermieden werden.

Ein weiterer Vorteil, welcher erfindungsgemäß erzielt werden kann, besteht darin, dass ein Anteil des zur Füllung des Blutschlauchsatzes erforderlichen Volumens der Priminglösung aufgrund der während des ersten Zeitraums anfänglich überhöht eingestellten Ultrafiltrationsrate bereits zu Beginn der Blutbehandlungssitzung über die Membran des Blutfilters aus dem Blutschlauchsatz in die Dialysierflüssigkeitskammer verschoben wird. Für diesen Anteil bedarf es später keiner - wie oben gezeigt fehler- und folgenbehafteten - Justierung der Bilanzierung. Die Plasma-Refilling-Rate, welche während der Behandlung als ein Gleichgewicht zwischen dem in die Gefäße wegen *refilling* eintretenden Flüssigkeitsvolumen und dem zeitgleich aufgrund der Ultrafiltration aus den Gefäßen entfernten Fluidvolumen angestrebt wird, kann aufgrund des erfindungsgemäßen abgegebenen Steuerbefehls bereits sehr früh nach Behandlungsbeginn erreicht werden. Das spätere Berücksichtigen von zusätzlich zu ultrafiltrierendem Volumen, was durch das zur Füllung des Blutschlauchsatzes erforderliche Volumen der Priminglösung nötig ist, kann vorteilhaft entfallen. Bei diesem Anteil kann es sich um jenen Anteil an Priminglösung handeln, welcher nicht bereits bei geschlossener venöser Patientenschlauchklemme über die Membran des Blutfilters aus dem Blutschlauchsatz in die Dialysierflüssigkeitskammer verschoben wurde.

Bei einem weiteren, auch als *bloody connection* bekannten Verfahren des Standes der Technik zum Vorbereiten des Blutschlauchsatzes zur Blutbehandlungssitzung wird nach dem Primen ein großer Anteil der im Blutschlauchsatz vorliegenden Priminglösung über den nicht mit dem Patienten verbundenen venösen Patientenschlauch in einen bereitgestellten Auffangbehälter verworfen. Abschließend wird der venöse Patientenschlauch manuell mit der venösen Patientennadel verbunden. Die Blutbehandlung kann beginnen. Anders als bei diesem bekannten Verfahren kann es erfindungsgemäß möglich sein, das manuelle Konnektieren des venösen Patientenschlauchs, welcher bis zu seinem Konnektieren beispielsweise aufgrund eines Kontakts mit dem Behälter zum Verwerfen der Priminglösung kontaminiert worden sein kann, unnötig zu machen. Der venöse Patientenschlauch kann nach seiner Entnahme aus einer sterilen Verpackung unmittelbar mit der venösen Patientennadel verbunden werden und dort verbleiben. Dies kann vorteilhaft zu einer verbesserten Hygiene und damit zu einer erhöhten Patientensicherheit beitragen.

Ferner bedarf es nicht der Bereitstellung eines Auffangbehälters, was Kosten hierfür sowie für spezielle *drain connectors* zur Verbindung des venösen Patientenschlauchs mit dem Auffangbehälter einsparen kann.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil stark vereinfachten Figuren gilt:

Fig. 1   zeigt schematisch vereinfacht eine erfindungsgemäße Vorrichtung zum Durchführen des offenbarten Verfahrens;

Fig. 2   zeigt schematisch als Flussdiagramm die Schritte des Verfahrens in einer ersten exemplarischen Ausführungsform;

Fig. 3   zeigt schematisch als Flussdiagramm die Schritte des Verfahrens in einer zweiten exemplarischen Ausführungsform;

**Fig. 4** zeigt beispielhaft den zeitlichen Verlauf des Verhältnisses von Fluid- zu Blut-Volumenstrom in der Blutkammer;

**Fig. 5** zeigt mögliche Zeitverläufe des Ultrafiltratflusses durch die Membran zwischen Blutkammer und Dialysierflüssigkeitskammer; und

**Fig. 6** zeigt einen möglichen zeitlichen Verlauf des Blutvolumens des Patienten während der Behandlungsdauer.

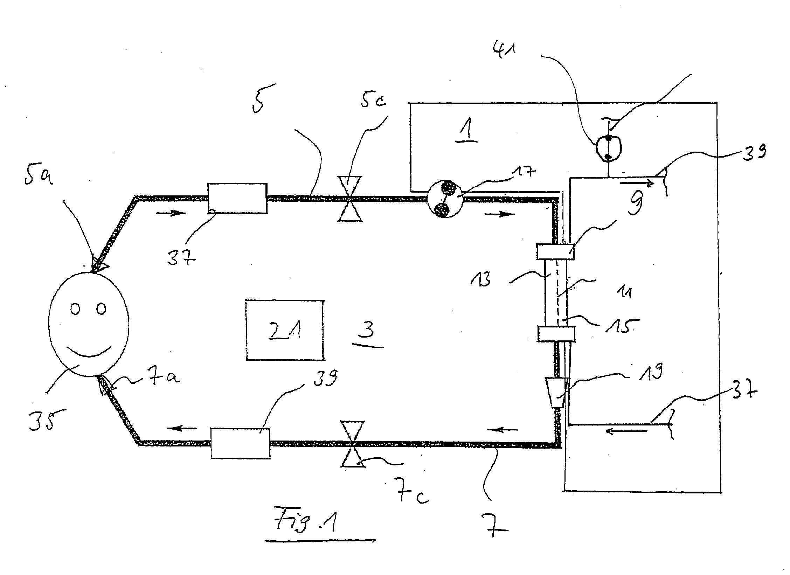

**Fig. 1** zeigt schematisch vereinfacht eine erfindungsgemäße Blutbehandlungsvorrichtung 1, welche verbunden ist mit einem Blutschlauchsatz 3. Der Blutschlauchsatz 3 weist einen arteriellen Abschnitt 5 mit einer arteriellen Nadel 5a und einer arteriellen Patientenschlauchklemme 5c auf. Der Blutschlauchsatz 3 weist ferner einen venösen Abschnitt 7 mit einer venösen Nadel 7a und einer venösen Patientenschlauchklemme 7c auf.

Der Blutschlauchsatz 3 weist einen Blutfilter 9 mit einer Membran 11, beispielsweise einer semi-permeablen Membran, auf. Die Membran 11 unterteilt den Blutfilter 9 in eine Blutkammer 13 und eine Dialysierflüssigkeitskammer 15.

Die Blutbehandlungsvorrichtung 1 weist eine Blutpumpe 17 auf. Der Blutschlauchsatz 3 weist eine venöse Tropfkammer 19 auf. Die Blutbehandlungsvorrichtung 1 weist eine Steuer- oder Regelvorrichtung 21 auf oder ist hiermit verbunden. Die Steuer- oder Regelvorrichtung 21 ist mit allen Komponenten der Blutbehandlungsvorrichtung 1 oder des Blutschlauchsatzes 3, oder beiden, verbunden, auf welche sie zur Ausführung des hierin offenbarten Verfahrens einzuwirken hat.

Sowohl die arterielle Nadel 5a als auch die venöse Nadel 7a sind mit dem Gefäßsystem eines Patienten 35 verbunden. Der Blutschlauchsatz 3 weist optional einen arteriellen Blutsensor 37', einen venösen Blutsensor 39' oder beide auf.

Der Blutfilter 9 ist mit einer Dialysierflüssigkeitszulaufleitung 37 und einer Dialysatablaufleitung 39 verbunden, welche beide nur abschnittsweise dargestellt sind. Eine mögliche Strömungsrichtung innerhalb dieser beiden Leitungen ist mit Pfeilen dargestellt.

Von der Dialysatablaufleitung 39 geht eine Stichleitung 41 ab. Diese steht mit einer Ultrafiltrationspumpe 43 in Wirkverbindung.

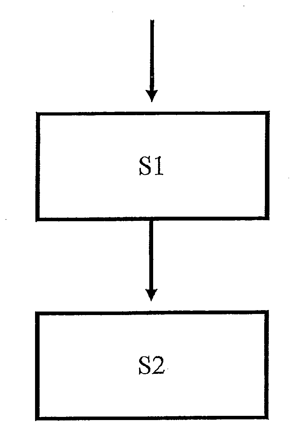

**Fig. 2** zeigt schematisch vereinfacht als Flussdiagramm die Schritte des offenbarten Verfahrens in einer ersten exemplarischen Ausführungsform.

In einem ersten Schritt S1 bleibt die venöse Patientenschlauchklemme 7c geschlossen. Die Blutpumpe 17 pumpt Blut über die arterielle Nadel 5a bei geöffneter arterieller Patientenschlauchklemme 5c bis in den Blutfilter 9 hinein. Dort tritt ein - insbesondere vorbestimmtes - Volumen aus der Blutkammer 13 in die Dialysierflüssigkeitskammer 15 über.

Ist das Volumen vorbestimmt, so kann es beispielsweise dem Füllvolumen des arteriellen Abschnitts 5 bis zum Blutfilter 9 (einschließlich oder ausschließlich dessen Volumens oder des Volumens seiner Blutkammer 15) entsprechen. Dieses Volumen kann optional vorab gemessen werden, beispielsweise mittels der Pumpenlaufzeit. Die Blutflussrate kann dabei der Ultrafiltrationsrate entsprechen, und umgekehrt.

In einem zweiten Schritt S2 wird die venöse Patientenschlauchklemme 7c geöffnet, sobald ein Sensor (nicht dargestellt) Blut im Blutfilter 9 oder in dessen Bereich erkennt.

Das Verfahren kann an dieser Stelle mit dem Öffnen der venösen Patientenschlauchklemme 7c beendet werden. Hieran kann sich das Behandlungsverfahren anschließen, welches nicht mehr Teil der vorliegenden Erfindung ist.

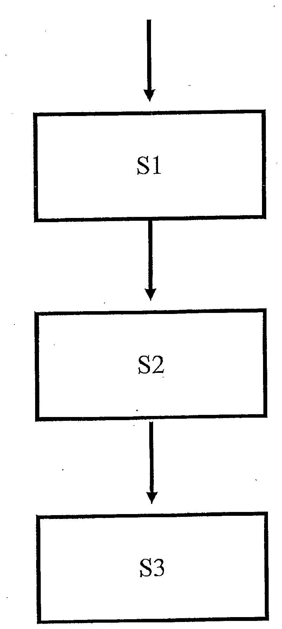

**Fig. 3** zeigt schematisch vereinfacht als Flussdiagramm die Schritte des offenbarten Verfahrens in einer zweiten exemplarischen Ausführungsform.

Die Schritte S1 und S2 entsprechen jenen, welche mit Bezug auf Fig. 2 beschrieben sind.

Hinzu kommt ein Schritt S3, in welchem ein Ausgeben eines Steuerbefehls an die Blutbehandlungsvorrichtung oder an einen Datenträger erfolgt. Der Steuerbefehl bewirkt, wenn er an die Blutbehandlungsvorrichtung gelangt, dass diese in einem ersten Zeitraum mit einer höheren Ultrafiltrationsrate filtriert als in einem nach dem ersten Zeitraum gelegenen zweiten Zeitraum. Dabei kann der erste Zeitraum nach Abschluss des Verfahrens liegen. Der erste Zeitraum kann somit zeitlich nach dem Abgabezeitpunkt des Steuerbefehls liegen.

Der Steuerbefehl kann bewirken, dass die Ultrafiltrationsrate innerhalb des ersten Zeitraums abnimmt.

Der Steuerbefehl kann bewirken, dass im ersten Zeitraum ein Volumen über die Membran hinweg filtriert wird, welches um das Fassungsvolumen der Blutkammer des Blutschlauchsets und des hiervon stromabwärts gelegenen Blutschlauchsatzes höher ist als ein Volumen, welches im zweiten Zeitraum über die Membran hinweg filtriert wird.

An den Schritt S3 kann sich wiederum das Behandlungsverfahren anschließen, welches ebenfalls nicht Teil der vorliegenden Erfindung ist.

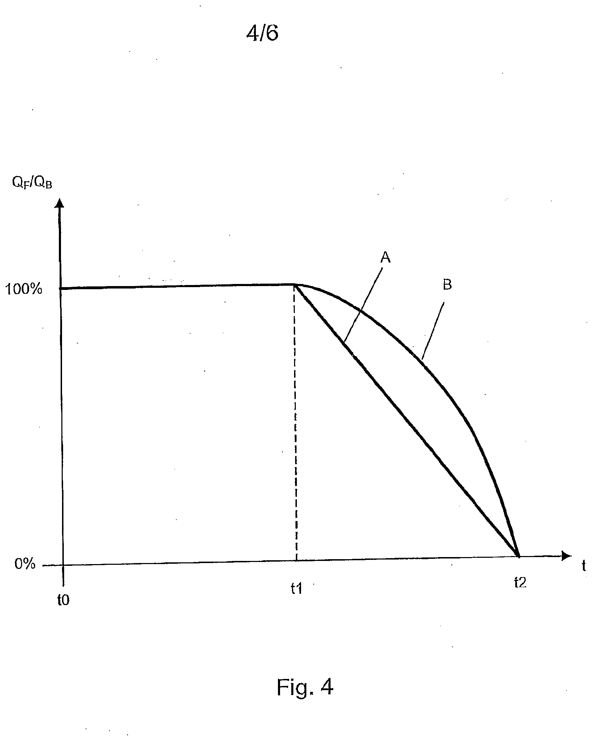

**Fig. 4** zeigt beispielhaft den zeitlichen Verlauf des Verhältnisses von Fluidvolumenstrom $Q_F$ (oder Ultrafiltrat-Volumenstrom $Q_{UF}$) zu Blut-Volumenstrom $Q_B$ in der Blutkammer.

In Fig. 4 gilt:

$Q_F/Q_B$:    Verhältnis von Fluid-Volumenstrom zu Blut-Volumenstrom.
100%: der Volumenstrom in der Blutkammer weist nur Fluid auf, kein Blut
0%: der Volumenstrom in der Blutkammer weist nur Blut auf, kein Fluid

Phase 1:    Zeitraum zwischen t0 und t1
Phase 2:    Zeitraum zwischen t1 und t2

A:    linearer Zeitverlauf von $Q_F/Q_B$ in der Phase 2, d.h. der Fluid-Volumenstrom nimmt kontinuierlich in der Blutkammer über der Zeit ab.

B:    nicht linearer Zeitverlauf von $Q_F/Q_B$ in der Phase 2; der Fluid-Volumenstrom nimmt in der Blutkammer zunächst weniger stark und dann zunehmend stärker ab.

**Fig. 5** zeigt mögliche Zeitverläufe des Ultrafiltratflusses $Q_{UF}$ durch die Membran zwischen Blutkammer und Dialysierflüssigkeitskammer.

In Fig. 5 gilt:

$Q_{UF}$:    Ultrafiltratfluss durch die Membran zwischen Blutkammer und Dialysierflüssigkeitskammer;

t3:    Beginn der Behandlung; t3 ist beispielsweise dann, wenn in der venösen Kammer mittels Sensor per Farbumschlag das Vorliegen von Blut erkannt wird;

t4:    Ende der Behandlung;

C:    nicht linear abnehmender Verlauf des durch das restliche Primingvolumen erhöhten Ultrafiltratflusses $Q_{UF}(t)$ über die gesamte Behandlungszeit;

D:    linear abnehmender Verlauf des erhöhten Ultrafiltratflusses von $Q_{UF}(t)$ über die gesamte Behandlungszeit; und

E:    Ultrafiltratfluss $Q_{UF}(t)$ ohne Berücksichtigung des durch das restliche Primingvolumen erhöhten Ultrafiltratflusses; der Verlauf E kann eine Sprungfunktion sein, wie in Fig. 5 gezeigt.

Das gesamte ultrafiltrierte Volumen, die sogenannte Volumenbilanz der Behandlung, kann gemäß folgender Formel ermittelt werden:

$$V_{UF,ges} = \int_{t3}^{t4} \left( Q_{UF}(t) + Q_{Priming}(t) \right) dt \quad ,$$

$V_{UF,ges}$:    gesamtes ultrafiltriertes Volumen im Zeitintervall t3 bis t4 (Behandlungszeit);

$Q_{UF}(t)$:    Ultrafiltratfluss durch die Membran zwischen Blutkammer und Dialysierflüssigkeitskammer während der Behandlungszeit, ohne Berücksichtigung des zusätzlichen Ultrafiltratflusses; das Integral entspricht der Fläche zwischen der x-Achse und "E"; und

$Q_{Priming}(t)$:    zusätzlicher Ultrafiltratfluss aufgrund des Rest-Primingvolumens; das Integral entspricht der Fläche zwischen "E" und "C" oder "D".

Der Zeitpunkt t3 kann mit dem Zeitpunkt t2 aus Fig. 4 zusammenfallen, muss aber nicht. Der Zeitpunkt t3 liegt jedenfalls aber nach t2.

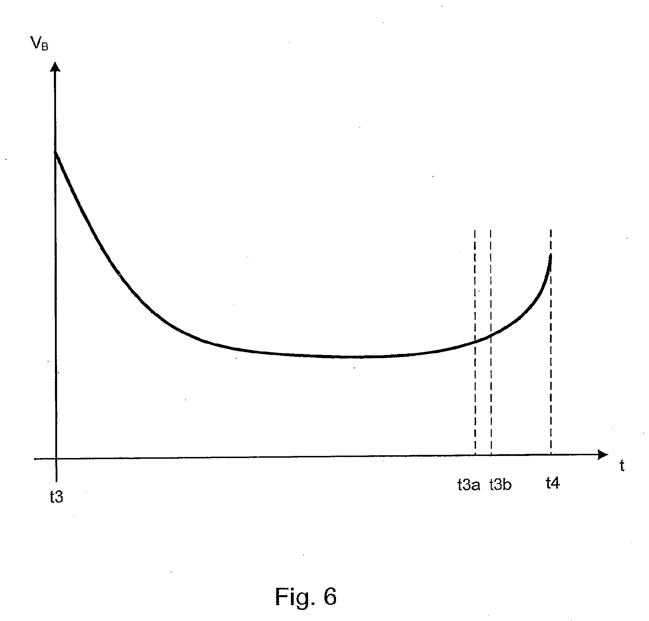

**Fig. 6** zeigt einen möglichen zeitlichen Verlauf des Blutvolumens des Patienten während der Behandlungsdauer.

Der Beginn der Behandlungssitzung ist der Zeitpunkt t3, das Ende der Zeitpunkt t4. Während der gesamten Behandlungssitzung wird dem Blut mittels Ultrafiltration Volumen entzogen.

Zum Zeitpunkt t3a wird ein Parameter als Maß für die Änderung des Plasmawasser-Anteils im Blut des Patienten bestimmt. Anschließend wird zum Zeitpunkt t3b oder einem sich t3b anschließenden oder t3b umfassenden Zeitraum ein zusätzliches Volumen (z.B. eine Kochsalzlösung, zugeführt als Infusion) mittels Infusion dem Blutvolumen des Patienten zugegeben. Das zusätzlich zugeführte Volumen kann beispielsweise einen erhöhten Volumenausgleich nach dem Ende der Behandlungssitzung innerhalb der beiden Flüssigkeitskompartimente des Patienten, dem extrazellulären Raum ECM und den Blutgefäßen, reduzieren oder diesem vorteilhaft entgegenwirken.

**Bezugszeichenliste**

| Bezugszeichen | Beschreibung |
|---|---|
| 1 | Blutbehandlungsvorrichtung |

(fortgesetzt)

| Bezugszeichen | Beschreibung |
|---|---|
| 3 | Blutschlauchsatz |
| 5 | arterieller Abschnitt |
| 5a | arterielle Nadel |
| 5c | arterielle Patientenschlauchklemme |
| 7 | venöser Abschnitt |
| 7a | venöse Nadel |
| 7c | venöse Patientenschlauchklemme |
| 9 | Blutfilter |
| 11 | Membran |
| 13 | Blutkammer |
| 15 | Dialysierflüssigkeitskammer |
| 17 | Blutpumpe |
| 19 | Venöse Tropfkammer |
| 21 | Steuer- oder Regelvorrichtung |
| 35 | Patient |
| 37 | Dialysierflüssigkeitszulaufleitung |
| 37' | arterieller Blutsensor |
| 39 | Dialysatablaufleitung |
| 39' | venöser Blutsensor |
| 41 | Stichleitung |
| 43 | Ultrafiltrationspumpe |

## Patentansprüche

1. Blutbehandlungsvorrichtung (1), aufweisend eine Steuer- oder Regelvorrichtung (21), programmiert, um automatisch ein Verfahren zum Steuern und/oder Regeln der Blutbehandlungsvorrichtung (1) im Zusammenwirken mit der Blutbehandlungsvorrichtung (1) auszuführen, wenn sie mit der Blutbehandlungsvorrichtung (1) verbunden ist,

   wobei die Blutbehandlungsvorrichtung (1) eine Blutpumpe (17) zum Fördern von Blut innerhalb eines extrakorporalen Blutschlauchsatzes (3) aufweist und mit dem Blutschlauchsatz (3) verbunden ist,
   wobei der Blutschlauchsatz (3) einen arteriellen Abschnitt (5) mit einer arteriellen Nadel (5a) und einer arteriellen Patientenschlauchklemme (5c), einen venösen Abschnitt (7) mit einer venösen Nadel (7a) und einer venösen Patientenschlauchklemme (7c), sowie einen zwischen den Abschnitten (5, 7) angeordneten Blutfilter (9) mit einer Membran (11), welche eine Blutkammer (13) von einer Dialysierflüssigkeitskammer (15) trennt, jeweils aufweist und/oder jeweils hiermit verbunden ist,
   wobei das Verfahren den Schritt umfasst:

   - Fördern von Fluid mittels einer Pumpe bei geschlossener venöser Patientenschlauchklemme (7c), aus dem Blutschlauchsatz (3) durch die Membran (11) hindurch in die Dialysierflüssigkeitskammer (15) und
   - Öffnen der venösen Patientenschlauchklemme (7c).

2. Blutbehandlungsvorrichtung (1) nach Anspruch 1, wobei das Verfahren den weiteren Schritt umfasst:

   - Fördern des Fluids, bis ein vorbestimmtes Volumen von Fluid in die Dialysierflüssigkeitskammer (15) übergetreten ist, oder bis wenigstens ein Sensorsignal das Erreichen von Blut an einer vorbestimmten Stelle detektiert.

3.  Blutbehandlungsvorrichtung (1) nach Anspruch 2, wobei das Verfahren den weiteren Schritt umfasst:

    - Reduzieren oder Stoppen des Fördern von Fluid, nachdem ein vorbestimmtes Volumen von Fluid in die Dialysierflüssigkeitskammer (15) übergetreten ist, oder wenigstens ein Sensorsignal das Erreichen von Blut an der vorbestimmten Stelle detektiert

4.  Blutbehandlungsvorrichtung (1) nach Anspruch 2 oder 3, wobei die vorbestimmte Menge an Volumen einem Volumen von Priminglösung entspricht, welches mittels der Pumpe aus dem arteriellen Abschnitt (5) durch die Membran (11) hindurch in die Dialysierflüssigkeitskammer (15) zugeführt wurde.

5.  Blutbehandlungsvorrichtung (1) nach einem der Ansprüche 1 bis 4, wobei die vorbestimmte Menge an Volumen einem Gesamtfassungsvermögen des Blutschlauchsatzes entspricht.

6.  Blutbehandlungsvorrichtung (1) nach einem der Ansprüche 1 bis 5, wobei das Verfahren weiter den Schritt umfasst:

    - Ausgeben eines Steuerbefehls an die Blutbehandlungsvorrichtung (1) derart, dass diese in einem, sich einem der Verfahren der vorangegangenen Ansprüchen zeitlich anschließenden, ersten Zeitraum mit einer höheren Ultrafiltrationsrate filtriert als in einem nach dem ersten Zeitraum gelegenen zweiten Zeitraum, wobei die venöse Patientenschlauchklemme (7c) im ersten und zweiten Zeitraum geöffnet ist.

7.  Blutbehandlungsvorrichtung (1) nach einem der Ansprüche 1 bis 5, wobei das Verfahren weiter den Schritt umfasst:

    - Ausgeben eines Steuerbefehls an die Blutbehandlungsvorrichtung (1) derart, dass diese in einem, sich dem Verfahren eines der vorangegangenen Ansprüchen zeitlich anschließenden, ersten Zeitraum mit einer niedrigeren Ultrafiltrationsrate filtriert als in einem nach dem ersten Zeitraum gelegenen zweiten Zeitraum, wobei die venöse Patientenschlauchklemme (7c) im ersten und zweiten Zeitraum geöffnet ist.

8.  Blutbehandlungsvorrichtung (1) nach Anspruch 6 oder 7, wobei der Steuerbefehl bewirkt, dass die Ultrafiltrationsrate innerhalb des ersten Zeitraums abnimmt.

9.  Blutbehandlungsvorrichtung (1) nach einem der Ansprüche 6 bis 8, wobei das Verfahren den Schritt umfasst:

    - Ausgeben eines Steuerbefehls an die Blutbehandlungsvorrichtung (1) derart, dass im ersten Zeitraum ein Volumen über die Membran (11) hinweg filtriert wird, welche um das Fassungsvolumen der Blutkammer (13) des Blutschlauchsets (3) und des venösen Abschnitts (7) höher ist als ein Volumen, welches im zweiten Zeitraum über die Membran (11) hinweg filtriert wird.

10. Blutbehandlungsvorrichtung (1) nach einem der vorangegangenen Ansprüche, wobei das Verfahren die weiteren Schritte umfasst:

    - Bestimmen eines Plasmawasser-Anteils im Blut wobei die Bestimmung wenigstens zu einem ersten Zeitpunkt, insbesondere zu Beginn der Behandlung (t3), und vorzugsweise auch zu einem zweiten Zeitpunkt (t4), insbesondere dem Ende der Behandlung durchgeführt wird;
    - Ausgeben eines Steuerbefehls an die Blutbehandlungsvorrichtung (1) derart, dass diese in einem Zeitraum, welcher zwischen dem ersten Zeitpunkt (t3) und dem zweiten Zeitpunkt (t4) liegt, mit einer ersten Ultrafiltrationsrate (UFR1) filtriert;
    - optionales Bestimmen des mittels der ersten Ultrafiltrationsrate (UFR1) gefilterten Volumens anhand wenigstens einer weiteren Bestimmung des Plasmawasser-Anteils im Blut zu einem zwischen dem ersten Zeitpunkt (t3) und dem zweiten Zeitpunkt (t4) liegenden dritten Zeitpunkt (t3a);
    - Zuführen von Fluid in den extrakorporalen Blutschlauchsatz (3) oder in das Gefäßsystem des Patienten (35) zu einem vierten Zeitpunkt (t3b), welcher ebenfalls zwischen dem ersten Zeitpunkt (t3) und dem zweiten Zeitpunkt (t4) liegt.

11. Blutbehandlungsvorrichtung (1) nach Anspruch 10, wobei das Verfahren den weiteren Schritt umfasst:

    - Zuführen von Fluid derart, dass zum zweiten Zeitpunkt (t4) der Plasmawasser-Anteil im Blut eine vorbestimmten Wert annimmt oder in einem vorbestimmt Wertebereich liegt.

**12.** Blutbehandlungsvorrichtung (1) nach Anspruch 11, wobei das Verfahren den weiteren Schritt umfasst:

- Bestimmen des Blutvolumens des Patienten (35) zum zweiten Zeitpunkt (t4).

**13.** Blutbehandlungsvorrichtung (1) nach einem der vorangegangenen Ansprüche, welche zur Dialyse, zur Akutdialyse, zur chronischen Dialyse oder zur Aquapherese ausgestaltet ist.

**Claims**

**1.** A blood treatment apparatus (1), comprising a control or regulating apparatus (21), programmed to automatically execute a method for controlling and/or regulating the blood treatment apparatus (1) in cooperation with the blood treatment apparatus (1) when connected with the blood treatment apparatus (1),

wherein the blood treatment apparatus (1) comprises a blood pump (17) for conveying the blood within an extracorporeal blood tubing set (3) and is connected to the blood tubing set (3),
wherein the blood tubing set (3) respectively comprises and/or is respectively connected with an arterial section (5) having an arterial needle (5a) and an arterial patient tubing clamp (5c), a venous section (7) having a venous needle (7a) and a venous patient tubing clamp (7c), as well as a blood filter (9) arranged between the sections (5, 7) having a membrane (11) which separates a blood chamber (13) from a dialysis fluid chamber (15), the method comprising the step of:

- conveying fluid from the blood tubing set (3) through the membrane (11) into the dialysis fluid chamber (15) by means of a pump when the venous patient tubing clamp (7c) is closed and
- opening the venous patient tubing clamp (7c).

**2.** The blood treatment apparatus (1) according to claim 1, the method comprising the further step of:

- conveying the fluid until a predetermined fluid volume has passed into the dialysis fluid chamber (15), or until at least a sensor signal detects that blood has reached a predetermined location.

**3.** The blood treatment apparatus (1) according to claim 2, the method comprising the further step of:

- reducing or stopping the conveying of fluid after a predetermined fluid volume has passed into the dialysis fluid chamber (15), or at least when a sensor signal detects that blood has reached the predetermined location.

**4.** The blood treatment apparatus (1) according to claim 2 or 3, wherein the predetermined volume amount corresponds to a priming solution volume supplied by means of the pump, from the arterial section (5), through the membrane (11), into the dialysis fluid chamber (15).

**5.** The blood treatment apparatus (1) according to anyone of claims 1 to 4, wherein the predetermined volume amount corresponds to a total volumetric capacity of the blood tubing set.

**6.** The blood treatment apparatus (1) according to anyone of claims 1 to 5, the method further comprising the step of:

- issuing a control instruction to the blood treatment apparatus (1) such that it filters, during a first time period following termination of the method according to anyone of the preceding claims, with a higher ultrafiltration rate during the first time period than during a second time period subsequent to the first time period, the venous patient tubing clamp (7c) being open during the first and second time periods.

**7.** The blood treatment apparatus (1) according to anyone of claims 1 to 5, the method further comprising the step of:

- issuing a control instruction to the blood treatment apparatus (1) such that it filters, during a first time period following termination of the method according to anyone of the preceding claims, with a lower ultrafiltration rate during the first period than during a second period subsequent to the first period, the venous patient tubing clamp (7c) being open during the first and second time periods.

**EP 3 021 886 B1**

8. The blood treatment apparatus (1) according to claim 6 or 7, wherein the control instruction causes the ultrafiltration rate to decrease within the first time period.

9. The blood treatment apparatus (1) according to anyone of claims 6 to 8, the method comprising the step of:

   - issuing a control instruction to the blood treatment apparatus (1) such that a volume is filtered through the membrane (11) during the first time period, said volume being higher than a volume filtered through the membrane (11) during the second time period, by the volume capacity of the blood chamber (13) of the blood tubing set (3) and the venous section (7).

10. The blood treatment apparatus (1) according to anyone of the preceding claims, the method further comprising the steps of:

    - determining a plasma water portion in the blood, the determination being executed at least at a first time point, in particular at the beginning of the treatment (t3), and preferably also at a second time point (t4), in particular at the end of the treatment;
    - issuing a control instruction to the blood treatment apparatus (1) such that it filters with a first ultrafiltration rate (UFR1) during a time period lying between the first time point (t3) and the second time point (t4);
    - optionally determining the volume filtered by means of the first ultrafiltration rate (UFR1) on the basis of at least one further determination of the plasma water portion in the blood at a third time point (t3a) lying between the first time point (t3) and the second time point (t4);
    - supplying fluid into the extracorporeal blood tubing set (3) or into the vascular system of the patient (35) at a fourth time point (t3b) lying also between the first time point (t3) and the second time point (t4).

11. The blood treatment apparatus (1) according to claim 10, the method further comprising the step of:

    - supplying fluid such that the plasma water portion in the blood assumes a predetermined value or lies within a predetermined range of values at the second time point (t4) .

12. The blood treatment apparatus (1) according to claim 11, the method further comprising the step of:

    - determining the blood volume of the patient (35) at the second time point (t4).

13. The blood treatment apparatus (1) according to anyone of the preceding claims, which is designed for dialysis, acute dialysis, chronic dialysis or aquapheresis.

**Revendications**

1. Un appareil de traitement du sang (1) comprenant un appareil de commande ou de régulation (21), programmé pour exécuter automatiquement un procédé de commande et/ou de régulation de l'appareil de traitement du sang (1) en coopération avec l'appareil de traitement du sang (1) lorsqu'il est connecté à l'appareil de traitement du sang (1),

   où l'appareil de traitement du sang (1) comprend une pompe à sang (17) pour acheminer le sang à l'intérieur d'un set extracorporel de tuyaux sanguins (3), et est relié au set de tuyaux sanguins (3),
   où le set de tuyaux sanguins (3) comprend respectivement, et/ou est relié respectivement à une section artérielle (5) ayant une aiguille artérielle (5a) ainsi qu'une pince de serrage artérielle de tuyau du patient (5c), une section veineuse (7) ayant une aiguille veineuse (7a) ainsi qu'une pince de serrage veineuse de tuyau du patient (7c), mais aussi un filtre sanguin (9) agencé entre les sections (5, 7) ayant une membrane (11) qui sépare une chambre à sang (13) d'une chambre à liquide de dialyse (15),
   le procédé comprenant l'étape consistant en:

     - l'acheminement de fluide depuis le set de tuyaux sanguins (3) au travers de la membrane (11) vers la chambre à liquide de dialyse (15) au moyen d'une pompe, lorsque la pince de serrage veineuse de tuyau du patient (7c) est fermée et
     - l'ouverture de la pince de serrage veineuse du tuyau du patient (7c).

2. L'appareil de traitement du sang (1) selon la première revendication, le procédé comprenant l'étape supplémentaire consistant en:

- l'acheminement du fluide jusqu'à ce qu'un volume prédéterminé de fluide ait atteint la chambre à liquide de dialyse (15), ou jusqu'à ce qu'au moins un signal de capteur détecte l'arrivée de sang à un endroit prédéterminé.

3. L'appareil de traitement du sang (1) selon la revendication 2, le procédé comprenant l'étape supplémentaire consistant en:

- la réduction ou l'arrêt de l'acheminement du fluide après qu'un volume prédéterminé de fluide ait atteint la chambre à liquide de dialyse (15), ou qu'au moins un signal de capteur ne détecte l'arrivée de sang à l'endroit prédéterminé.

4. L'appareil de traitement du sang (1) selon la revendication 2 ou 3, où la quantité de volume prédéterminée correspond à un volume de solution d'amorçage amené depuis la section artérielle (5) au travers de la membrane (11) vers la chambre à liquide de dialyse (15) au moyen de la pompe.

5. L'appareil de traitement du sang (1) selon l'une quelconque des revendications 1 à 4, où la quantité de volume prédéterminée correspond à une capacité volumétrique totale du set de tuyaux sanguins.

6. L'appareil de traitement du sang (1) selon l'une quelconque des revendications 1 à 5, le procédé comprenant l'étape supplémentaire consistant en:

- l'émission d'un ordre de commande à l'appareil de traitement du sang (1) de telle sorte que celui-ci, au cours d'une première période de temps postérieure au procédé de l'une quelconque des revendications précédentes, filtre avec un taux d'ultrafiltration plus élevé au cours de la première période de temps qu'au cours d'une seconde période de temps postérieure à la première période de temps, la pince de serrage veineuse de tuyau du patient (7c) étant ouverte au cours de la première ainsi que de la seconde période de temps.

7. L'appareil de traitement du sang (1) selon l'une quelconque des revendications 1 à 5, le procédé comprenant l'étape supplémentaire consistant en:

- l'émission d'un ordre de commande à l'appareil de traitement du sang (1) de telle sorte que celui-ci, au cours d'une première période de temps postérieure au procédé de l'une quelconque des revendications précédentes, filtre avec un taux d'ultrafiltration plus faible au cours de la première période de temps qu'au cours d'une seconde période de temps postérieure à la première période de temps, la pince de serrage veineuse de tuyau du patient (7c) étant ouverte au cours de la première ainsi que de la seconde période de temps.

8. L'appareil de traitement du sang (1) selon la revendication 6 ou 7, où l'ordre de commande provoque une diminution du taux d'ultrafiltration au cours de la première période de temps.

9. L'appareil de traitement du sang (1) selon l'une quelconque des revendications 6 à 8, le procédé comprenant l'étape consistant en:

- l'émission d'un ordre de commande à l'appareil de traitement du sang (1) de telle sorte qu'un volume filtré au travers de la membrane (11) au cours de la première période de temps soit supérieur à un volume filtré au travers de la membrane (11) dans la seconde période de temps de la capacité volumique de la chambre à sang (13) du set de tuyaux sanguins (3) et de la section veineuse (7).

10. L'appareil de traitement du sang (1) selon l'une quelconque des revendications précédentes, le procédé comprenant les étapes supplémentaires consistant en:

- la détermination d'une teneur en eau plasmatique dans le sang, la détermination étant effectuée au moins à un premier instant, notamment au début du traitement (t3), et de préférence également à un second instant (t4), notamment à la fin du traitement;
- l'émission d'un ordre de commande à l'appareil de traitement du sang (1) de telle sorte que celui-ci filtre avec un premier taux d'ultrafiltration (UFR1) au cours d'une période de temps située entre le premier instant (t3) et le second instant (t4);
- la détermination facultative du volume filtré au moyen du premier taux d'ultrafiltration (UFR1) sur la base d'au moins une autre détermination de la teneur en eau plasmatique dans le sang à un troisième instant (t3a) situé entre le premier instant (t3) et le second instant (t4);
- l'alimentation en fluide dans le set extracorporel de tuyaux sanguins (3) ou dans le système vasculaire du patient (35) à un quatrième instant (t3b) également situé entre le premier instant (t3) et le second instant (t4).

11. L'appareil de traitement du sang (1) selon la revendication 10, le procédé comprenant l'étape supplémentaire consistant en:

- l'alimentation en fluide de sorte qu'au second instant (t4), la teneur en eau plasmatique dans le sang atteigne une valeur prédéterminée ou se situe dans une plage de valeurs prédéterminée.

12. L'appareil de traitement du sang (1) selon la revendication 11, le procédé comprenant l'étape supplémentaire consistant en:

- la détermination du volume sanguin du patient (35) au second instant (t4).

13. L'appareil de traitement du sang (1) selon l'une quelconque des revendications précédentes, qui est conçu pour la dialyse, la dialyse aiguë, la dialyse chronique ou l'aquaphérèse.

Fig.1

S1

S2

```
           │
           ▼
      ┌──────────────┐
      │              │
      │      S1      │
      │              │
      └──────────────┘
           │
           ▼
      ┌──────────────┐
      │              │
      │      S2      │
      │              │
      └──────────────┘
           │
           ▼
      ┌──────────────┐
      │              │
      │      S3      │
      │              │
      └──────────────┘
```

4/6

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009018664 A1
- US 4670152 A
- DE 19655226 B4
- WO 2006002685 A1

**In der Beschreibung aufgeführte Nicht-Patentliteratur**

- **SCHÖNWEIß, GÜNTHER.** Dialysefibel. Verlagsgesellschaft mbH, 2006, vol. 2, 481-482
- **HÖRL, W. H. ; WANNER C.** Dialyseverfahren in Klinik und Praxis. Georg Thieme Verlag, 2003, 210